# EUROPEAN PATENT SPECIFICATION

(11) **EP 2 735 340 B1**
(45) Date of publication and mention of the grant of the patent: **14.11.2018**
(21) Application number: 13447017.8
(22) Date of filing: 18.11.2013
(51) Int. Cl.: A61Q 19/00, A61K 8/97, A61K 8/27, A61K 8/66, A61Q 19/06, A61K 8/92, A61K 8/02, A61K 8/19, A61K 8/20, A61K 8/96

(54) **Method and compositions to remove toxins from the body.**
Verfahren und Zusammensetzungen, um Giftstoffe aus dem Körper zu entfernen.
Methode et compositions pour enlever des toxines du corps.

(30) Priority: 21.11.2012 BE 201200782
(43) Date of publication of application: 28.05.2014
(73) Proprietor: Angrave, Wendy, 2800 Mechelen (BE); Stuyck, Patrick, 2800 Mechelen (BE)
(72) Inventor: Angrave, Wendy, 2800 Mechelen (BE); Stuyck, Patrick, 2800 Mechelen (BE)

(56) References cited:
- WO-A1-2011/121539
- WO-A2-01/45661
- FR-A- 2 842 102
- US-A- 4 717 735
- US-A1- 2003 188 378
- SCHMID D., SCHÜRCH C., MÜLLER S., ZÜLLI F.: "Second generation antioxidants from cress sprouts", SÖFW-JOURNAL, vol. 13, no. 3, 2007, pages 2-6, XP002715265,
- REULAND DANIELLE ET AL: "Upregulation of heme oxygenase-1 through activation of Nrf2 by the phytochemicals in Protandim", THE FASEB JOURNAL, FEDERATION OF AMERICAN SOCIETIES FOR EXPERIMENTAL BIOLOGY, US, vol. 24, 1 April 2010 (2010-04-01), XP009178293, ISSN: 0892-6638

## Description

### TECHNICAL FIELD

The invention relates to the field of cosmetic methods of removing toxins and waste substances from the body for the purpose of (i) improving the skin and the structure of the underlying fatty tissue, (ii) improving body contours, (iii) reducing body volume, (iv) reducing the thickness of the subcutaneous fatty tissue layer, (v) treating cellulite and (vi) increasing the effectiveness of diet and exercise for weight loss.

### BACKGROUND ART

The skin is the largest organ of the body and plays an important part in protecting the body from undesirable substances. However it is not completely impermeable. Nava Dayan, Cosmetics & Toiletries magazine (2005) Vol. 120 No. 6 pp 67-76 describes the routes of penetration of substances into the skin. The same routes can facilitate extraction of substances out of the skin.

Dirt and excess sebum accumulated on the skin surface or in the pores can disturb the natural barrier function of the skin and cause skin problems such as acne, blocked pores and uneven skin tone. Methods and compositions to remove these substances include cleansing compositions, mechanical cleansers (such as cloths and brushes), mechanical exfoliators and chemical exfoliators.

Chemical substances in the deeper skin layers can damage lipids, and interact with cellular macromolecules such as proteins and DNA. As a result, cell metabolism is impaired, resulting in fatigued, dull skin, loss of skin strength and elasticity, accelerated ageing of the skin and increased risk of irritation, allergy, sensitivity, inflammation and skin problems such as psoriasis, eczema and pigmentation. Exposure of the skin to UV radiation or other solar or electromagnetic radiation influences the metabolism of toxicants in the body and increases levels of harmful free radicals or reactive oxygen species. (Ernest Hodgson, A Textbook of Modern Toxicology 3rd edition, J. Wiley & Sons Inc, Hoboken NJ USA pp237-238)

Substances can also enter the body via the penetration routes of the skin, via ingestion or via inhalation. They are then metabolized and either stored in adipose tissue or excreted.

The main exposure routes and the fate of toxicants in the body have been documented. Some occupations, and also factors such as lifestyle, diet and genetic disposition involve increased risk of exposure to toxicants. Such factors can be linked to the accumulation of specific toxins and/or their metabolised intermediates in the subcutaneous adipose tissue. For example, exposure to benzene from tobacco smoke, petrol fumes, exhaust fumes or some medicines leads to accumulation of toxic metabolites such as quinone in the body. Ernest Hodgson, A Textbook of Modern Toxicology 3rd edition, J. Wiley & Sons Inc, Hoboken NJ USA pp4-5 and pp33-73 describes exposure routes and the sources of toxins such as air pollutants, water pollutants, soil pollutants, occupational toxicants, industrial toxicants, toxic metals, agricultural chemicals, food additives, food contaminants, microbial toxins, solvents, drugs; combustion products and cosmetics. Many publications from the European Agency for Safety and Health at Work (EU-OSHA) describe occupational risks including information sheet E-facts 34, 23/09/2008, "Risk assessment for Hairdressers"; E-facts 41, 17/10/2008, "Cleaners and dangerous substances" and Factsheet 40, 18/06/2003, "Skin sensitisers".

In the subcutaneous adipose tissue, waste substances and toxins may be stored within the fat cells themselves, or in the interstitial fluid which surrounds the fat cells. Stored toxins can cause swelling of the skin and subcutaneous adipose tissue layers, leading to blockages in the lymphatic system and weakening of the tissue structure. Left untreated, toxins accumulate and the effects become increasingly visible as increased body volume, decreased firmness of the skin and subcutaneous adipose tissue, uneven skin surface and uneven skin tone. This accumulation of toxins is a major factor in the formation of protruding fat nodules which become visible at the surface, a condition referred to as cellulite or orange peel skin.

Ernest Hodgson, A Textbook of Modern Toxicology 3rd edition, J. Wiley & Sons Inc, Hoboken NJ USA p204 describes how chemicals stored in lipids tend to be largely immobile and difficult to release from the adipose tissue. The accumulation of waste substances and toxins can increase with age but is largely dependant on factors including lifestyle, dietary habits, gender, occupation, hormones, genetic disposition, disease, medication and medical treatments.

The presence of waste substances and toxins in the subcutaneous adipose tissue impairs the effectiveness of diet and exercise for weight-loss. (A.Tremblay, C.Pelletier, E.Doucet and P.Imbeault, International Journal of Obesity (2004) Vol. 28, pp936-939).

Cosmetic methods to remove toxins from the body work by either (i) extracting the substances out of the body via the skin or (ii) stimulating the body's own elimination system utilising the routes via the liver, the kidneys or the lungs.

Breakdown of the subcutaneous adipose tissue increases the amount of toxicants circulating in the body. Breakdown of the subcutaneous adipose tissue occurs during weight loss, but can also be induced by cosmetic methods including cavitation, ultra sound, massage, connective tissue massage, fat-freezing, vacuum massage, infra-red treatment, radio frequency treatment, laser fat reduction and mesotherapy, or by cosmetic ingredients and/or nutritional supplements to stimulate lipolysis.

Toxins circulating freely in the body can be detrimental to health, and methods which break down fat can therefore be inadvisable or may involve an unacceptably high risk for a large number of people. J.Chevrier, É.Dewailly, P.Ayotte, P.Mauriège, J-P Després and A.Tremblay, International Journal of Obesity (2000) Vol. 24, No.10, pp1272-1278 describes for example how weight loss in obese individuals increases subcutaneous adipose tissue concentrations of potentially toxic pollutants. Many cosmetic treatments to break down fat cannot be practiced on overweight individuals.

Well known cosmetic methods for extracting toxicants and waste substances out of the body via the skin include using the adsorbent and/or absorbent properties of substances and colloid & surface phenomena in treatments such as baths, body wraps and masks. Other methods of extracting toxicants and waste substances out of the body via the skin include inducing transpiration and extraction by reverse iontophoresis.

US 4 717 735 describes a body wrap method which achieves a measurable reduction in body volume by removing toxins to reduce swelling of the skin and subcutaneous adipose tissue. The result occurs without weight loss, indicating that the method does not rely on water loss or sweating. A body wrap of this type is commercially available from Silky Oaks BVBA.

DE4017172 describes the use of squalane and squalene for the removal of toxins and waste products through the skin.
EP2429481 describes methods and compositions for improving skin and body appearance.
FR2968213 describes a combination of ingredients for slimming.
SI1392335 describes active ingredients for the treatment of cellulite

The uptake and release of toxins at a cellular level in the body has been the subject of much research. Three phases have been identified in the metabolism of toxins. Phase I involves the transformation of a toxic lipophilic compound to form a reactive molecule. The resulting substance may be much more harmful to the body than the original substance. Phase II involves the addition of a water-soluble group to the molecule, making it hydrophilic and easier to eliminate. Phase III is a pumping mechanism which facilitates removal of these substances from the cells into the surrounding interstitial fluid.

Phase I and phase II are regulated by enzymes. Substances which induce phase I enzymes are regarded as harmful to health unless the resulting substances are immediately processed by phase II enzymes and eliminated from the body.

Many cosmetic and nutritional ingredients support the detoxification processes. Deficiency of such substances can impair the efficiency of the detoxification processes. These substances are often incorporated into cosmetic compositions and described in marketing and sales literature as 'anti-oxidants', 'co-factors' or ingredients to 'shield or protect from oxidative stress'.

A smaller number of substances can actively increase cellular detoxification by either inducing a phase II enzyme or by increasing phase III activity. The substrates of each phase II enzyme are documented. For example, NADPH quinone oxidoreductase acts on the quinone 1,4-Benzoquinone to form the hydroquinone 1,4-Benzenediol. Ernest Hodgson, A Textbook of Modern Toxicology 3rd edition, J. Wiley & Sons Inc, Hoboken NJ USA p111-148 and D.J.Liska, Alternative Medicine Review (1998) Vol 3, No. 3 pp187-198 describe the detoxification enzymes and their substrates.

### TECHNICAL PROBLEM

Toxins stored in the subcutaneous adipose tissue are known to be immobile and difficult to remove. There is a long sought-after need for improved methods to remove toxins from the skin and from the subcutaneous tissue.

Despite the many treatments currently available, increasing numbers of people suffer from overweight or are dissatisfied with cellulite and body and skin appearance. There is a need for improved cosmetic methods to address these problems.

Methods that break down fatty tissue increase the toxic load on the elimination processes of the body. There is a need for such methods which are safer than current methods and which can be practiced on a wider group of people.

Increased toxic load increases the amount of toxins circulating in the body and is detrimental to health. Reabsorption of toxins into the fatty tissue is also likely. This leads to results which are disappointing and less long-lasting than expected.

Ingredients which increase cellular detoxification also increase the quantity of toxins circulating in the body. Toxins circulating freely can be detrimental to health. There is a need for improved removal of toxins from the body without increasing the toxic load.

### SUMMARY OF THE INVENTION

It is a purpose of the invention to provide an alternative methode for eliminating toxins from the skin and to improve the results of cosmetic treatments by combining a method of extracting toxins with the application of a cosmetic composition comprising ingredients to stimulate the release of toxins from the fat cells into the interstitial fluid.

Current extracting methods work on waste products and toxins in the skin and in the interstitial fluid of the subcutaneous adipose tissue but they cannot access toxins within the fat cells. This is due to the nature of the cell membrane. Toxins trapped within the cells remain there until conditions are such that they can be acted on by the detoxification phases II and III.

It is therefore a purpose of the invention to provide a method which increases phase II and/or phase III detoxification. This increases the quantity of toxins in the interstitial fluid which can be removed.

It is a further purpose of the invention to provide a method of improving cellulite and body contours by reducing the amount of toxins in the subcutaneous adipose tissue without increasing the amount of toxins circulating freely in the body.

During weight loss, the release of toxins from the fat cells slows down the metabolism, making further weight loss more difficult. It is therefore a further purpose of the invention to provide a method of removing toxins from the fat cells during weight loss so that the efficiency of a weight loss plan is increased.

When phase II is stimulated, more toxins are in a form suitable for removal by phase III activity and/or an extracting treatment. However, when phase II detoxification is stimulated without further attention to the removal of toxins, the body's own elimination system can become overloaded. This also happens when lipolysis is induced. It is therefore a further purpose of the invention to provide an improved method of removing toxins from the body without increasing the toxic load on the body.

Methods to break down subcutaneous fatty tissue destroy the fat cell wall and release toxins into the interstitial fluid. These toxins are released without first undergoing the normal cellular detoxification processes. This makes them difficult to remove, and therefore the toxic load on the body increases. It is a further purpose of the invention to improve methods of treating the subcutaneous fatty tissue by increasing the detoxification processes and the removal of toxins via the skin.

Methods of determining results are described below. The results show an unexpected synergy.

### DETAILED DESCRIPTION

The present invention is defined in the claims.

The words "comprising/comprise/comprises" as used herein have an inclusive, open meaning and do not exclude further non-specified components, elements or steps. The words "including/include/includes" and "such as" do not exclude further non-specified components, elements or steps. The Dutch words "omvattend"/"omvatten"/"omvat" and "bevattend"/"bevatten"/ "bevat" as used in the original description have the English meaning "comprising/comprise/comprises".
All documents cited in the description are herein incorporated in their entirety by reference.

The subject of the invention is generally a human but the invention can also be applied to an animal. The term "person" as used herein means the subject of the invention. "Body" means any part or parts of the body including hands, face, feet, hair, scalp and nails.

The term "general health" as used herein means the health of the subject in terms of hormones, medication, medical treatments, disease and genetic disposition.

The term "cosmetically acceptable" as used herein means legally permissible for use in cosmetics.
The terms "toxins", "toxicants" and "waste substances" are used interchangeably and as used herein mean all toxicants in the body or on the surface of the body, including free radicals and reactive oxygen species.
The term "to improve cellulite" and "to treat cellulite" are used interchangeably and as used herein means to improve the appearance of cellulite and/or to prevent cellulite and/or to delay the onset of cellulite.
The terms "extracting method" and "extracting treatment" are used interchangeably and as used herein mean any non-invasive method to remove toxins via the skin from the skin and skin surface, from the pores and from the subcutaneous adipose tissue.
The term "cellular detoxification" as used herein includes phase II detoxification and phase III detoxification.
The term "cellular detoxification inducer" as used herein is a substance capable of increasing cellular detoxification by (i) increasing phase II detoxification by inducing a phase II detoxification enzyme and/or by (ii) increasing phase III detoxification.

Cosmetic ingredients are referred to using their INCI name except where a commonly used name is preferable for understanding.

Examples are given as illustrations of the invention but should not be construed as limiting the invention.

In one embodiment the extracting treatment is a body wrap.
Body wrap treatments involve the application of a composition to the whole body or to a part of the body by either applying the composition and then textile to the skin, or by applying textile to the skin that has been soaked in the composition or a solution or suspension thereof.

The composition comprises one or more cosmetically acceptable ingredients selected from clay, peat, mud, silt, minerals, inorganic salts, plant extracts, algae, squalane, squalene or a combination thereof.

The term 'clay' as used herein includes cosmetic ingredients such as those with the INCI names bentonite, montmorillonite, smectite clay, kaolin, argilla, argilla extract, aluminum silicate, calcium magnesium silicate, magnesium aluminum silicate, Canadian colloidal clay, clay, clay extract, clay minerals, hectorite, Elguea clay, Heilmoor clay, Hokkaido Akan clay, illite, Manicouagan clay, marine clay, lava clay, Moroccan lava clay, Ghassoul, Palau white clay, sea clay extract, solum fullonum (Fuller's Earth), Tanakura clay, zeolite, loess, sodium magnesium silicate, magnesium silicate, attapulgite and/or sepiolite.

The term 'minerals' as used herein includes cosmetic ingredients such as such as those with the INCI names dolomite, mudstone powder, pumice, tourmaline, chalk, talc, pegmatite, perlite, nephrite powder, zinc zeolite, silica and zinc oxide.

The term 'peat' as used herein includes cosmetic ingredients such as such as those with the INCI names peat, peat moss, peat moss extract, peat water and moor.

The term 'mud' as used herein includes cosmetic ingredients such as such as mud, sea mud, sea mud extract, black mud, alluvial mud, limus, salt mine mud, thermal mud and sulphur mud.

The term 'silt' as used herein includes cosmetic ingredients such as such as those with the INCI names silt, silt extract and sea silt.

The term 'inorganic salts' as used herein includes cosmetic ingredients such as such as those with the INCI names magnesium sulfate, magnesium chloride, sodium chloride, zinc oxide, magnesium carbonate, sodium hexametaphosphate, sodium borate, sodium citrate, magnesium oxide, magnesium silicate, sodium bicarbonate, sodium sesquicarbonate, sodium carbonate, sodium perborate, sea salt, sea salt extract, sea water extract and maris sal, or a solution of one or more inorganic salts such as ingredients with the INCI name sea water or maris aqua.

The term 'algae' as used herein includes cosmetic ingredients such as such as those with the INCI names algae, algae extract, algae oligosaccharides and algae ferment.

The term 'plant extract' as used herein includes cosmetic ingredients such as starch, flour, fruit seed powder, fruit peel powder, colloidal oatmeal, seed flour and aloe vera.

The form of the textile may a bandage, dressing, gauze, compress, garment, or support stocking. The textile may be elastic or of a fixed form. The textile may be for one use only or reusable. One more pieces of textile can be used to cover the whole body or part of the body. The subject may optionally be covered using synthetic clothing, foil, film, blanket, towelling or garment. During the body wrap treatment the subject may rest or be subjected to other treatments including but not limited to electrical muscle stimulation, vibration massage, infra-red heating, sauna, infra-red sauna, massage, connective tissue massage, vacuum massage, hydro-massage, lymph drainage and/or exercise.

In another embodiment the extracting method is a body wrap using bandages soaked in a solution or suspension comprising one or more ingredients from the groups clay, inorganic salts and/or mud as described above. In a preferred embodiment, the extracting method is a body wrap as described in example 1.

In another embodiment, the extracting method is a mask. The form of the mask may be a suspension, gel, serum, cream, lotion, paste, liquid, wax, powder or patch. The mask may be a clay mask, peel-off mask, exfoliating mask, cleansing mask, self-heating mask, seaweed mask, oxygenating mask, aromatherapy mask, collagen mask, paraffin mask, body pack or facial pack. The mask may be applied to the whole body or to a part of the body. The mask comprises one or more ingredients selected from clay, peat, mud, silt, inorganic salts, plant extract, algae, squalane and/or squalene
In another embodiment the extracting method is a clay mask. A clay mask comprises one or more ingredients selected from the clay group described above. The clay mask may be applied to the whole body or to a part of the body.
Example 2 gives examples of masks which can be used in the present invention.

In another embodiment, the extracting method is the use of bath salts. The bath salts comprise one or more ingredients selected from the group of inorganic salts as described above. The form may be a powder, crystals or tablets. A solution or suspension of the bath salts is used to soak the whole body or part of the body in a bath, footbath or other suitable container or apparatus or in an environment where the salt or salt composition is present. In addition ingredients may be added for the purpose of effervescence, foaming, colour, fragrance, purifying, skin moisturising and/or water softening. In addition one or more ingredients may be added to the bath salts from the groups clay, plant extracts and algae described above. An example of a bath salts formulation which can be used in the invention is given in Example 3.

In another embodiment the extracting method is the use of a mud composition as a mud bath. The whole body or part of the body can be bathed or packed in the mud composition in a bath, footbath or other suitable container or apparatus or in an environment where the mud composition is present. The mud composition may be pure mud or a composition comprising one or more ingredients from the groups mud, clay, peat and/or silt as described above.

In another embodiment the extracting treatment is a bath soak. The treatment involves bathing in water to which an infusion or extract has been added. The whole body or part of the body can be bathed or soaked in a bath, footbath or other suitable container. Examples of ingredients which can be used as an infusion or extract include essential oils, algae, vegetable oil and ingredients with the INCI names sea clay extract, salt extract, plant extract, peat extract, mud extract, silt extract and sea salt extract.

The extracting treatment can also be the inducement of transpiration to force toxins and waste products out of the skin. Transpiration may be induced by using heat, exercise, electrical stimulation, mechanical stimulation or occlusion, or a combination hereof.

In one embodiment the extracting treatment is the inducement of transpiration by heat application using one or more methods such as dry sauna, steam sauna, infra-red sauna, far-infra-red sauna, infra-red heater, sauna belt, multiple sauna belts, application of UV radiation or steam bath or a combination hereof.

In another embodiment transpiration is induced by exercise. The exercise may be self exerted or forced using an electrical or mechanical apparatus. The apparatus may be a massage apparatus, vibrating apparatus, vacuum massage apparatus, toning table or electrical muscle stimulation, or a combination thereof. The electrical stimulation method may be galvanic current, faradic current, microcurrent, high frequency current or a combination thereof.
In another embodiment transpiration is induced by occlusion of the skin. The whole body or part of the body may be occluded using a material such as but not limited to plastic, vinyl, foil, film or paraffin wax, or a mud pack or mud bath, textile, garment or combination thereof. An example is given below.
In another embodiment, the extracting method is reverse iontophoresis. The reverse iontophoresis may be induced using an electrical apparatus using galvanic current, faradic current, microcurrent or a combination thereof. The reverse iontophoresis can also be induced using an ionising bath, ionising footbath or one or more ionising patches. The reverse iontophoresis may be applied to the whole body or to one or more parts of the body.

The cellular detoxification composition is a topical composition comprising a base composition and at least one active ingredient to increase cellular detoxification by (i) increasing phase II detoxification by inducing a phase II detoxification enzyme and/or by (ii) increasing phase III detoxification.

The cellular detoxification composition is prepared in the form suitable for application on the skin through rubbing, spraying, patch, compress, textile, bath soak or body wrap composition. The cellular detoxification composition can be in the form of a cream, gel, serum, lotion, ointment, mask, aqueous solution, mixture, suspension, emulsion, oil or anhydrous preparation. Skin penetration and product efficiency can be increased by the encapsulation of one or more ingredients, or by the use of iontophoresis. Types of encapsulation include the use of liposomes, microspheres, microemulsions, coated liposomes, phospholipid vectorisation and micelles. Iontophoresis can be carried out using galvanic current, faradic current, microcurrent or a combination thereof. The preferable current is microcurrent because it increases cellular levels of ATP, which aids phase III detoxification.

The base composition is a cosmetically acceptable formula and comprises one or more components selected from the group containing water, oil, emulsifiers, surfactants, humectants, thickeners, conditioners, stabilisers, solubilisers, preservatives, fragrance, pigments, dyes, emollients, film formers, sunscreens, pH adjusters, and antioxidants for the protection of oil components. A person skilled in the art will be able to create a suitable base formula or use example formulas provided in textbooks. Examples of base formulas are given below.

Active ingredients are added to the base composition taking into account the effect of the chosen concentration on the performance, safety and stability of the end product. The form in which the ingredient is available is also taken into account. Hydrophilic preparations of active ingredients may be added to the water phase of the formulation during processing. Lipophilic preparations of active ingredients may be added to the oil phase of the formulation during processing. Alternatively, and especially in the case of heat sensitive or fragile ingredients, they may be added by gentle stirring at the end of processing. The base composition may also be the composition which is used for an extraction treatment.

Cellular detoxification can be increased by inducing phase II enzyme activity. In one embodiment the cellular detoxification composition comprises one or more active ingredients to induce a phase II detoxification enzyme. The ability of a substance to induce phase II enzyme activity can be determined using commercially available PCR (Polymerase Chain Reaction) array kits which represent the genes encoding the major classes of phase II enzymes. Cells are cultured in a medium with and without the test substance. After incubation the cells are harvested, the total RNA is extracted, converted into cDNA and then analysed using the PCR cycling program. Such a kit is commercially available from www.sabosciences.com.

Phase II detoxification enzymes include the enzyme group Glucuronosyltransferases which regulate Glucuronide conjugation reactions; the enzyme groups Sulfotransferases and Sulfatases which regulate sulfate conjugation reactions; the enzyme group methyltransferases which regulate methylation; the enzyme group Glutathione S-transferases which regulate glutathione conjugation reactions; N-acetyl transferase which regulates acetylation; the enzyme group amino acid transferases which regulate amino acid conjugation reactions; quinone reductase and heme oxygenase.

In one embodiment, the cellular detoxification composition comprises at least one substance to induce a phase II enzyme. Such substances include isothiocyanates, diphenols, thiocarbamates, 1,2-dithol-3-thiones and indole-3-carbinol. Sulforaphane for example is an isothiocyanate that can be synthesised or produced from plant sources including broccoli, cauliflower, kale, brussel sprouts, cress, garden cress sprouts, rocket, cabbage, diakon, kohlrabi, garden cress, mustard, radish, turnip, watercress, crambe and seeds and sprouts from the Brassicaceae family. Sulforaphane induces phase II enzymes including NADPH:quinone oxidoreductase 1, Heme oxygenase 1 and Thioredoxin reductase 1.

Xymenynic acid induces enzymes including Gluthathione peroxide 2 and Gluthathione transferase.
Turmerone, present in the cosmetic ingredient Curcuma Longa root extract, induces enzymes including Thioredoxin reductase, Gluthathione peroxide and NADPH:quinone oxidoreductase. Other cosmetic ingredients providing substances with the ability to induce phase II enzymes include those with INCI names Tsuga Canadensis (hemlock) leaf extract, Limnanthes alba seed oil, Ellagic acid, Schizandra Chinensis fruit oil and citrus triterpenoids.

The PCR array tests can be used to optimise properties of the cellular detoxification composition such as active ingredient concentrations and pH.

Cellular detoxification can also be increased by increasing phase III detoxification.

Substances with the ability to increase phase III detoxification include encapsulated magnesium chloride or encapsulated forms of salts, sea salts or other salt mixtures comprising magnesium. Encapsulation is important for such ingredients. The most suitable encapsulation method is a liposome coated with polysaccharides (Example 1). The ability of a cosmetic ingredient to increase phase III detoxification can be measured by P-glycoprotein (P-gp) activity assessment using a commercially available luminescent Pgp ATPase assay (www.promega.com).
Other substances with this ability include hypericin and epicatechin. Hypericin is found in the cosmetic ingredient Hypericum Perforatum Extract. Epicatechin is found in the cosmetic ingredient Camellia Sinensis Leaf Extract.

In one embodiment, the cellular detoxification composition comprises one or more ingredients to induce phase III detoxification.
In a preferred embodiment, the cellular detoxification composition comprises a gel-cream base, at least one ingredient to induce a phase II enzyme and at least one ingredient to increase phase III detoxification activity. The preferred ingredients are (i) sulforaphane from garden cress sprouts to induce phase II enzymes and (ii) encapsulated sea salt extract to increase phase III activity.

Additionally, the cellular detoxification composition may comprise ingredients to aid the elimination of toxins and increase results by (i) elevating levels of reduced glutathione, (ii) increasing antioxidant activity, (iii) strengthening the blood vessels of the vascular system, (iv)increasing blood flow, (v) stimulating lipolysis and/or (vi) inhibiting adipogenesis.

Glutathione is an important intracellular antioxidant, existing in a reduced (GSH) and oxidised state (GSSG). The amount of GSH in relation to GSSG or to total glutathione can be correlated with antioxidant activity. The ability of an ingredient to elevate cellular levels of GSH can be measured by using a commercially available Total Glutathione Assay kit. Substances with this ability include alpha lipoic acid, N-acetylcysteine, glutamine, glycine, tocopherol, alkylresorcinols and flavonoids. Cosmetic ingredients providing substances to elevate cellular levels of GSH levels include Malachite extract, Sophora Japonica Flower extract, Angelica Archangelica Root, Silybin, Grapefruit extract, Olea Europaea Fruit extract, citrus fruit extract and bran extract.

Antioxidant activity can be increased by adding ingredients which provide or increase the availability of co-factors. The ability of an ingredient to do this can be determined using a commercially available Total Antioxidant Capacity (TAC) assay kit (www.cellbiolabs.com). Substances with this ability include Polyphenols such as resveratrol; flavonoids including soy isoflavones, lignans and stilbenes, proanthocyanidin en quercetin; uric acid, lipoic acid, carotenoids, ferulic acid, caffeic acid, Coenzyme Q10, vitamin A, vitamin B, vitamin C and vitamin E. Cosmetic ingredients providing substances which increase antioxidant activity include those with the INCI names Genistein, Citrus Grandis Fruit Extract, Camellia Sinensis Leaf Extract, Vitis Vinifera Seed Extract, Silybin, Daucus Carota Sativa Root Extract, Solanum Lycopersicum Extract, Coffea Robusta Seed Extract, Punica Granatum Seed Extract, Niacinamide and Pinus Pinaster Bark Extract.

Elimination of toxins can be increased by adding ingredients to strengthen the blood vessels. The ability of an ingredient to do this can be determined by in vitro evaluation of the receptorial activity of the test ingredient on collagen synthesis. Substances with this ability include rutin, asiaticoside, silicic acid and citrus flavonoids. Cosmetic ingredients providing substances to strengthen the vascular system include those with the INCI names Centella Asiatica Extract, Equisetum Arvense Extract, Solidago Virgaurea Extract, Arnica Montana Flower Extract, Vaccinium Macrocarpon Fruit Extract and Hedera Helix Extract.

Elimination of toxins can also be aided by adding ingredients which increase blood flow. The ability of an ingredient to do this can be determined by blood flow quantification of microcirculation in a certain area using a commercially available laser Doppler blood flow monitor (www.perimed-instruments.com). Substances known to increase blood flow include ruscogenin, escin, forskolin and citrus flavonoids. Cosmetic ingredients providing substances to increase blood flow include those with the INCI names Ruscus Aculeatus Root Extract, Coleus Forskohlii Root Extract, Vaccinium Myrtillus Fruit/Leaf Extract and Aesculus Hippocastanum Extract.

The results obtained by the cellular detoxification composition can be increased by adding ingredients with the ability to induce lipolysis. This can be determined using a commercially available Lipolysis Assay Kit (www.zen-bio.com). Fat cells are mixed with the test ingredient and stirred for a fixed time, typically 2 hours. Increase in lipolysis is then determined by measurement of the amounts of glycerol and/or free fatty acids and comparison with a control substance. Substances with this ability include xanthines such as caffeine, theophylline and theobromine; isoproterenol, tripeptide-3, tripeptide-1, L-carnitine, synephrine and flavonoids. Cosmetic ingredients providing substances to induce lipolysis include those with the INCI names Genistein, Paullinia Cupana Fruit Extract, Citrus Aurantium Amara Peel, Cynara Scolymus (Artichoke) Bud Extract, Fucus Vesiculosus Extract, Algae Extract, Magnolia Biondii Bark Extract and Cola Nitida Seed Extract.

The results obtained by the cellular detoxification composition can be increased by adding ingredients with the ability to inhibit adipogenesis. This can be determined by incubating preadipocytes for a fixed period of time, typically 7 days, then staining the intracellular lipids to enable quantification and comparison with a control. Substances with this ability include fisetin, frambinone, aspartame, resveratrol, rosmarinic acid and tripeptides of the sequence Val-Tyr-Pro or Val-Thr-Leu. Cosmetic ingredients providing substances to inhibit adipogenesis include Astragalus Membranaceus Root Extract, Nephelium Longana Seed Extract, Vitis Vinifera Seed Extract, Rosmarinus Officinalis Extract and Nigella Sativa Seed Oil.

Some occupations involve increased exposure to specific toxicants and can lead to increased accumulation of such toxicants or their metabolites in the skin and the adipose tissue. Such occupations include chemical plant worker, textile worker, medical chemist, industrial chemist, radiologist, hairdresser, nail-technician and occupations involving exposure to electromagnetic radiation, exhaust fumes, petrol fumes or polluted air. Lifestyle, dietary habits, gender and general health can also influence the type of toxicants and their accumulation in the body. In one embodiment, at least one cellular detoxification inducer is selected according to the occupation, lifestyle, dietary habits, age, gender and/or the general health of the subject and the resulting specific toxins to be removed from the body. The substrates of each phase II enzyme are known and are matched to the specific toxins to be removed. One or more active ingredients can then be selected according to their ability to induce the required enzyme. This selection can be done using a PCR array test kit.

The extracting treatment can be applied to the whole body or to one or more parts of the body. The cellular detoxification composition is applied one or more times to the whole body or to one or more parts of the body. The preferred manner of combining the cellular detoxification composition with an extracting method is described in Example 1.

The efficiency of the invention can be determined by assessing one or more results such as body volume reduction, reduction in the thickness of the skin and/or subcutaneous adipose tissue, changes in skin firmness, changes in skin pigmentation, changes in cellulite, changes in skin roughness and changes in efficiency of a weight loss plan. The efficiency of the invention can also be determined by subjective evaluation by the test subject.

Body volume reduction can be measured by using a tape measure to measure circumference at fixed points on the body. The points are marked using pen marks to ensure that the position of the measurements is consistent. The measurements are carried out by a person sufficiently skilled and practised to ensure a consistent tension of the tape measure. Body volume reduction can also be determined by using digital photography or optical 3D photography methods.

Reduction in the thickness of the skin and subcutaneous adipose tissue can be measured using skinfold calipers at chosen fixed points on the body. The points are marked using pen marks to ensure that the position of the measurements is consistent. The measurements are carried out by a person sufficiently skilled and practised to ensure consistent measurement.

Reduction in skin thickness can be measured using commercially available ultra sound imagery equipment.

Changes in skin firmness can be determined by using commercially available ultra sound imagery equipment to measure skin density. Changes in skin pigmentation can be visually assessed, or determined by using digital photography or a chromameter.

Changes in cellulite can be measured using thermographic film. This is used to grade cellulite and to grade circulation in the skin and subcutaneous adipose tissue layers. Changes in cellulite can also be measured by visual assessment of digital photos. Changes in cellulite can also be measured using visual cellulite grading by a skilled person.

Changes in cellulite can also be determined by changes in skin smoothness, roughness or thickness. Changes in skin roughness and smoothness can be measured using optical 3D methods.

Changes in the efficiency of a weight loss plan can be determined by measuring loss of lean mass and fat mass over fixed periods of time. Another way to evaluate changes is to determine the basal metabolic rate at regular intervals. This is done by measuring oxygen consumption and CO2 production. Changes in the metabolic rate and its deviation from control values can be used to evaluate changes in the efficiency of a weight loss plan.

### EXAMPLES

### Example 1.

The preferred embodiment of the extracting treatment is a body wrap using bandages soaked in a body wrap composition comprising bentonite, magnesium sulfate, magnesium chloride, sodium chloride, zinc oxide and water. The most preferred embodiment is a body wrap of this type that is commercially available from Silky Oaks BVBA and sold under the name Silky Oaks® Contour Wrap. The result is measured in terms of reduction in body volume. The measurements are taken manually using a tape measure. The preferred embodiment of the cellular detoxification composition is a gel-cream composition comprising the active ingredients (i) sulforaphane from garden cress sprout extract to induce phase II enzymes and (ii) encapsulated sea salt extract to increase phase III detoxification.
Formula (INCI, wt%): Aqua 89.1; Helianthus annuus seed oil 3.0; Sodium acrylates copolymer/Hydrogenated polyisobutene/Phospholipids/Polyglyceryl-10 Stearate/Helianthus annuus seed oil 4.4; Lepidium Sativum sprout extract 0.01; Glycerin 0.27; Lecithin 0.06; water/Sea salt extract/Propanediol/Phospholipids/Stearoyl Inulin 3.15; Rosmarinus Officinalis Extract 0.01; preservative qs.

The cellular detoxification composition is applied to the skin twice daily for a period of 30 days, except for the days when an extraction treatment is carried out. Application is by gentle massage movements until the composition is absorbed in to the skin. After 10 days, and spread over a period of 3 weeks, 3 body wrap extracting treatments are carried out. Measurements are taken before the first application of the cellular detoxification composition. The measurements are repeated before and after each extracting treatment.

### Example 2.

Mask composition for body pack extracting treatment:
Formula (INCI, wt%): Aqua 47.4; Magnesium Aluminum Silicate 2; Xanthan Gum 0.5; Glycerin 5; Sodium Phytate 0.1; Silt 45; Preservative qs.
Clay mask composition for extracting treatment:
Formula (INCI, wt%): Aqua 82.6; Bentonite 11.5; Zinc oxide 4.8; Phenoxyethanol/Methylparaben/Ethylparaben/Butyl paraben/Propylparaben/Isobutylpara ben 1.1
The mask is applied to the skin after the application of a cellular detoxification composition. After 10-60 (preferably 20) minutes the mask is removed with water.

### Example 3.

### Bath salts as extracting treatment

Formula (INCI, wt%): Magnesium sulfate 80.0; sodium bicarbonate 15.0; bentonite 5.0. A subject rests for 10-60 (preferably 20) minutes in a warm (preferably 37°C - 45°C) solution of bath salts in water.

### Example 4.

A weight loss plan includes diet and exercise to lose 10kg over the course of a 10 week period. The cellular detoxification composition is applied twice daily to tummy, hips, thighs and upper arms twice daily for the 10 week period, or until the target weight is reached. Extracting treatments consisting of a bath salt treatment followed by a steam sauna treatment are carried out weekly the 10 week period, or until the target weight is reached.

### Example 5.

Bandages which have been soaked in a solution comprising the cellular detoxification composition, are used to cover the parts of the body to be treated. The whole body or at least the parts of the body being treated are covered to give an extracting treatment by occlusion. After a rest period of 10-60 minutes, preferably 30 minutes, the bandages are removed.

### Example 6.

### Base formulas.

Base formula Cream (INCI, wt%): Glyceryl Stearate 4.0; Stearic acid 16.0; Ceteareth-12 3.0; Octyldodecanol 3.1; Paraffinum liquidum 3.1; Triethanolamine 0.5; Aqua 69.3;
Phenoxyethanol/Methylparaben/Ethylparaben/Butylparaben/Propylparaben/Isobutylpara ben 0.7; fragrance 0.3.

Base formula Serum-lotion (INCI, wt%): Aqua 92.2; Glyceryl Caprylate 1.1; Sodium acrylates copolymer/Lecithin 3.3; Helianthus annuus seed oil 3.4; preservative qs;
fragrance qs.

Base formula Gel (INCI, wt%): Aqua 95.25; Acrylates/C10-30 Alkyl Acrylate
crosspolymer 0.71; Glycerin 4.04; water soluble dye qs; preservative qs; fragrance qs;
Sodium Hydroxide (to pH5.5)

Base formula Tonic (INCI, wt%):
Aqua to 100; Glycerin 10; preservative qs; fragrance qs; water soluble dye qs.

### Example 7.

Cellular detoxification composition (oil serum) (INCI, wt%): Simmondsia Chinensis Seed Oil 45.5; Helianthus Annuus Seed Oil 42.5, Squalane 9.0; Caprylic/Capric Triglyceride 2.1; Xymenynic acid 0.5; Nigella Sativa Seed Oil 0.2; Tocopherol 0.2. Cellular detoxification composition (gel) (INCI, wt%): Aqua 90.3; Acrylates/C10-30 Alkyl Acrylate crosspolymer 0.68; Glycerin 3.95; Aqua/Malachite extract 3.5; Lepidium Sativum sprout extract 0.01; Lecithin 0.05; Aqua/sea salt
extract/propanediol/phospholipids/stearoyl inulin 1.51; Sodium Hydroxide (to pH5.5);
preservative qs.

Cellular detoxification composition (tonic) (INCI, wt%): Aqua 90.1; Butylene Glycol 3.0; Glycerin 3.0; Sodium Phytate 0.1; Aqua/sea salt
extract/propanediol/phospholipids/stearoyl inulin 3.0;
Phenoxyethanol/Methylparaben/Ethylparaben/Butylparaben/Propylparaben/Isobutylpara ben 0.8.

Cellular detoxification composition (for removal of toxins due to an occupation or lifestyle involving petrol fume exposure; Polymerase Chain Reaction array test shows inducement of NADPH quinone oxidoreductase) (INCI, wt%): Aqua 88.41; Sodium Phytate 0.1; Glyceryl Caprylate 1.04; Sodium acrylates copolymer/Lecithin 3.12;
Helianthus annuus seed oil 2.75; Curcuma Longa root extract 1.0;
Lepidium Sativum sprout extract 0.01; Glycerin 0.27; Lecithin 0.06; water/sea salt
extract/propanediol/phospholipids/stearoyl inulin 3.23; Rosmarinus Officinalis Extract 0.01; preservative qs; fragrance qs.

### Example 8 Test protocol example.

Measurements are taken on the left and right legs. These measurements are referred to below as M0.
A cellular detoxification composition is applied to the left leg during the test period. An equal amount of the base composition, without active ingredients to increase cellular detoxification, is applied to the right leg during the same test period. The compositions are applied a fixed number of times each day during the test period.

After the test period, the measurements are repeated. These measurements are referred to below as M1.
An extracting treatment is then carried out. The measurements are repeated after the extracting treatment and referred to below as M2.
Changes shown by comparing the right leg measurements M1 and M0 represent changes due to factors other than those of the invention. These changes are referred to below as R0.

Changes due to the cellular detoxification composition alone are determined by comparing the left leg measurements M1 and M0 and then subtracting R0.

Changes due to the extracting treatment alone are determined by comparing the right leg measurements M2 and M1.
Changes due to the combination of a cellular detoxification composition and extracting treatment are determined by comparing the left leg measurements M2 and M0 and then subtracting R0.
The changes in comparison with the original measurements may also be expressed as a percentage change. The test period can be repeated to provide measurements after further extraction treatments.
Tests were carried out on cellular detoxification compositions and extracting treatments as described. Cellular detoxification compositions with (i) a formula comprising at least one ingredient to induce a phase II detoxification enzyme, (ii) a formula comprising at least one ingredient to increase phase III detoxification activity and (iii) a formula comprising both at least one ingredient to induce a phase II detoxification enzyme and at least one ingredient to increase phase III detoxification activity were tested.
All results show an unexpected and synergistic effect in that the results of the combined extracting method and cellular detoxification composition are better than the sum of the results of using the extracting method and cellular detoxification composition separately.

### INDUSTRIAL APPLICATION

The invention increases the effectiveness of current cosmetic methods for removing toxins from the skin and subcutaneous adipose tissue. The results are improved by the synergistic action of (i) making more toxins available for removal and (ii) by removing the newly available toxins more efficiently from the body so that the body is less taxed by the detoxification process. The invention can be applied to achieve improved results in the areas of (i) body volume reduction, (ii) treatment of cellulite, (iii) improving and firming of the skin and subcutaneous adipose tissue (iv) body contour improvement, (v) removal of toxins from the skin and body and (vi) weight loss.

## Claims

1. A cosmetic method for improved removal of toxins from the skin and subcutaneous fatty tissue comprising
(i) an extracting treatment selected from (ia) body wrap, mask, clay mask, bath salts, mud bath and bath soak, (ib) transpiration induced by heat, exercise, electrical stimulation, mechanical stimulation, occlusion or reverse iontophoresis and
(ii) the application of a topical composition comprising at least one cellular detoxification inducer, wherein said cellular detoxification inducer is (iia) a substance to induce a phase II detoxification enzyme selected from isothiocyanates, diphenols, thiocarbamates, 1,2-dithol-3-thiones, indole-3-carbinol, Sulforaphane, Brassicaceae extract, Lepidium Sativum (Garden cress) sprout extract, Xymenynic acid, Curcurma Longa root extract, Schizandra Chinensis fruit oil, Hemlock Leaf extract, Limnanthes alba seed oil, citrus triterpenoids or Ellagic acid, or wherein said cellular detoxification inducer is (iib) a substance to increase phase III detoxification selected from encapsulated magnesium chloride, encapsulated salt, encapsulated sea salt, sea salt, encapsulated salt extract, encapsulated sea salt extract, Hypericum perforatum extract or Camellia Sinensis leaf extract.

2. A method according to any previous claim wherein at least one ingredient of said composition is encapsulated by at least one technique selected from liposomes, microspheres, microemulsion, coated liposomes, phospholipid vectorisation or micelles.

3. A method according to any previous claim wherein said extracting treatment is a body wrap using bandages soaked in a body wrap composition comprising at least one ingredient selected from clay, bentonite, magnesium chloride, magnesium sulphate, sodium chloride or zinc oxide.

4. A method according to any previous claim wherein said composition is applied using iontophoresis by a current type selected from microcurrent, galvanic current or faradic current.

5. A method according to any previous claim wherein said cellular detoxification composition comprises at least one additional ingredient selected from (i) alpha lipoic acid, N-acetylcysteine, glutamine, glycine, tocopherol, alkylresorcinols flavonoids, Malachite extract, Sophora Japonica Flower extract, Angelica Archangelica Root, Silybin, Grapefruit extract, Olea Europaea Fruit extract, citrus fruit extract, bran extract, polyphenols, flavonoids, stilbenes, proanthocyanidin, quercetin; uric acid, lipoic acid, carotenoids, ferulic acid, caffeic acid, Coenzyme Q10, vitamin A, vitamin B, vitamin C, vitamin E, Genistein, Citrus Grandis Fruit Extract, Camellia Sinensis Leaf Extract, Vitis Vinifera Seed Extract, Silybin, Daucus Carota Sativa Root Extract, Solanum Lycopersicum Extract, Coffea Robusta Seed Extract, Punica Granatum Seed Extract, Niacinamide, Pinus Pinaster Bark Extract, rutin, asiaticoside, silicic acid, citrus flavonoids, Centella Asiatica Extract, Equisetum Arvense Extract, Solidago Virgaurea Extract, Arnica Montana Flower Extract, Vaccinium Macrocarpon Fruit Extract, Hedera Helix Extract, ruscogenin, escin, forskolin, Ruscus Aculeatus Root Extract, Coleus Forskohlii Root Extract, Vaccinium Myrtillus Fruit/Leaf Extract, Aesculus Hippocastanum Extract, xanthines, isoproterenol, tripeptide-3, tripeptide-1, L-carnitine, synephrine, Paullinia Cupana Fruit Extract, Citrus Aurantium Amara Peel, Cynara Scolymus (Artichoke) Bud Extract, Fucus Vesiculosus Extract, Algae Extract, Magnolia Biondii Bark Extract, Cola Nitida Seed Extract, fisetin, frambinone, aspartame, resveratrol, rosmarinic acid tripeptides of the sequence Val-Tyr-Pro, tripeptides of the sequence Val-Thr-Leu, Astragalus Membranaceus Root Extract, Nephelium Longana Seed Extract, Vitis Vinifera Seed Extract, Rosmarinus Officinalis Extract or Nigella Sativa Seed Oil.

6. A method according to any previous claim wherein said method is for a purpose selected from (i) body volume reduction, (ii) the treatment of cellulite, (iii) improving and firming of the skin and subcutaneous adipose tissue (iv) body contour improvement, (v) the removal of toxins from the skin and body, (vi) improving weight loss, (vii) improving a body wrap method, (viii) improving cosmetic slimming methods.

7. A method according to any previous claim wherein at least one active ingredient to induce a phase II detoxification enzyme is chosen according to a factor selected from occupation, lifestyle, dietary habits, age, gender or general health of the subject.

8. A composition for use in therapeutic methods for the removal of toxins from the skin and subcutaneous fatty tissue, wherein said cellular detoxification composition comprises (i) at least one ingredient to induce a phase II detoxification enzyme selected from isothiocyanates, diphenols, thiocarbamates, 1,2-dithol-3-thiones, indole-3-carbinol, Sulforaphane, Brassicaceae extract, Lepidium Sativum (Garden cress) sprout extract, Xymenynic acid, Curcurma Longa root extract, Schizandra Chinensis fruit oil, Hemlock Leaf extract, Limnanthes alba seed oil, citrus triterpenoids or Ellagic acid; and (ii) at least one ingredient to increase phase III detoxification selected from encapsulated magnesium chloride, encapsulated salt, encapsulated sea salt, sea salt, encapsulated salt extract, encapsulated sea salt extract, Hypericum perforatum extract or Camellia Sinensis leaf extract.

## Patentansprüche

1. Ein kosmetisches Verfahren zur verbesserten Entfernung von Giftstoffen aus der Haut und Unterhautfettgewebe umfassend (i) eine Extraktionsbehandlung ausgewählt aus (ia) Körperpackung, Maske, Tonmaske, Badesalz, Schlammbad, Bad, (ib) Transpiration induziert durch Hitze, Bewegung, elektrische Stimulation, mechanische Stimulation, Okklusion oder umgekehrte Iontophorese und (ii) die Anwendung einer topischen Zusammensetzung, umfassend mindestens aus eine zellulärer Entgiftungsinduktor, wobei der zelluläre Entgiftungsinduktor ist (iia) eine Substanz zur Induktion eines Entgiftungsenzyms der Phase II, ausgewählt aus Isothiocyanate, Diphenole, Thiocarbamate, 1,2-Dithol-3-thione, Indole-3-Carbinol, Sulforaphan, Brassicaceae-Extrakt, (INCI) Lepidium Sativum sprout extract (Garten Kresse), Xymeninsäure, (INCI) Curcurma Longa root extract, (INCI) Schizandra Chinensis fruit oil, Hemlock-Blatteextrakt, (INCI) Limnanthes alba seed oil, Citrus-Triterpenoide oder (INCI) Ellagic acid, oder wobei der zelluläre Entgiftungsinduktor ist (iib) eine Substanz zur Induktion eines Entgiftungsenzyms der Phase III, ausgewählt aus Eingekapseltes Magnesiumchlorid, eingekapseltes Salz, eingekapseltes Meersalz, Meersalz, eingekapseltes Salz-Extrakt, eingekapseltes Meersalz-Extrakt, (INCI) Hypericum perforatum extract oder (INCI) Camellia Sinensis leaf extract.

2. Verfahren nach einem der vorhergehenden Ansprüche, wobei mindestens ein Bestandteil der Zusammensetzung wird mit mindestens eine Technik eingekapselt, ausgewählt aus Liposomen, Mikrokügelchen, Mikroemulsion, beschichtete Liposomen, Phospholipid-Vektorisierung oder Micellen.

3. Verfahren nach einem der vorhergehenden Ansprüche, wobei die Extraktionsbehandlung eine Körperpackung ist, wobei Bandagen verwendet werden, die in einer Körperpackung Zusammensetzung getränkt sind, die mindestens einen Bestandteil umfasst, der ausgewählt ist aus Ton, Bentonit, Magnesiumchlorid, Magnesiumsulfat, Natriumchlorid oder Zinkoxid.

4. Verfahren nach einem der vorhergehenden Ansprüche, wobei die Zusammensetzung unter Verwendung von Iontophorese durch einen Stromtyp ausgewählt aus Mikrostrom, galvanischem Strom oder faradischem Strom aufgebracht wird.

5. Verfahren nach einem der vorhergehenden Ansprüche, wobei die zelluläre Entgiftung Zusammensetzung mindestens einen zusätzlichen Bestandteil umfasst, der ausgewählt ist aus Alpha-Liponsäure, N-Acetylcystein, Glutamin, Glycine, Tocopherol, Alkylresorcinole Flavonoide, (INCI) Malachite extract, (INCI) Sophora Japonica Flower extract, (INCI) Angelica Archangelica Root, (INCI) Silybin, (INCI) Grapefruit extract, (INCI) Olea Europaea Fruit extract, Zitrusfruchtextrakt, Kleie-Extrakt, Polyphenole, Flavonoide, Stilbene, Proanthocyanidine, (INCI) Quercetin; (INCI) Uric acid, Liponsäure, Carotenoide, (INCI) Ferulic acid, (INCI) Caffeic acid, Coenzym Q10, Vitamin A, Vitamin B, Vitamin C, Vitamin E, (INCI) Genistein, (INCI) Citrus Grandis Fruit Extract, (INCI) Camellia Sinensis Leaf Extract, (INCI) Vitis Vinifera Seed Extract, (INCI) Silybin, (INCI) Daucus Carota Sativa Root Extract, Solanum Lycopersicum Extrakt, (INCI) Coffea Robusta Seed Extract, (INCI) Punica Granatum Seed Extract, (INCI) Niacinamide, (INCI) Pinus Pinaster Bark Extract, (INCI) Rutin, (INCI) Asiaticoside, Kiezelsäure, Zitrusflavonoide, (INCI) Centella Asiatica Extract, (INCI) Equisetum Arvense Extract, (INCI) Solidago Virgaurea Extract, (INCI) Arnica Montana Flower Extract, (INCI) Vaccinium Macrocarpon Fruit Extract, (INCI) Hedera Helix Extract, (INCI) Ruscogenin, (INCI) Escin, Forskolin, (INCI) Ruscus Aculeatus Root Extract, (INCI) Coleus Forskohlii Root Extract, (INCI) Vaccinium Myrtillus Fruit/Leaf Extract, (INCI) Aesculus Hippocastanum Extract, Xanthine, Isoproterenol, (INCI) Tripeptide-3, (INCI) Tripeptide-1, L-Carnitin, (INCI) Synephrine, (INCI) Paullinia Cupana Fruit Extract, (INCI) Citrus Aurantium Amara Peel, (INCI) Cynara Scolymus (Artichoke) Bud Extract, (INCI) Fucus Vesiculosus Extract, Algenextrakt, (INCI) Magnolia Biondii Bark Extract, (INCI) Cola Nitida Seed Extract, (INCI) Fisetin, Frambinon, (INCI) Aspartame, (INCI) Resveratrol, (INCI) Rosmarinic acid, Tripeptide der Sequenz Val-Tyr-Pro, Tripeptide der Sequenz Val-Thr-Leu, (INCI) Astragalus Membranaceus Root Extract, (INCI) Nephelium Longana Seed Extract, (INCI) Vitis Vinifera Seed Extract, (INCI) Rosmarinus Officinalis Extract oder (INCI) Nigella Sativa Seed Oil.

6. Verfahren nach einem der vorhergehenden Ansprüche, wobei das Verfahren für einen Zweck ist, ausgewählt aus (i) Körpervolumenreduzierung, (ii) die Behandlung von Cellulite, (iii) Verbesserung und Festigung der Haut und Unterhautfettgewebe (iv) Verbesserung der Körperkontur, (v) die Entfernung von Giftstoffen aus Haut und Körper, (vi) Verbesserung des Gewichtsverlustes, (vii) Verbesserung einer Körperwickelmethode, (viii) Verbesserung der kosmetischen Schlankheitsmethoden.

7. Verfahren nach einem der vorhergehenden Ansprüche, wobei mindestens ein Wirkstoff zur Induktion eines Entgiftungenzyms der Phase II nach einem Faktor von Besetzung, Lebensstil, Ernährungsgewohnheiten, Alter, Geschlecht oder allgemeine Gesundheit des Subjekts ausgewählt gewählt wird.

8. Eine Zusammensetzung zur Verwendung in therapeutischen Verfahren um Giftstoffe aus der Haut und Unterhautfettgewebe zu entfernen, wobei die zelluläre Entgiftung Zusammensetzung umfasst (i) mindestens ein Bestandteil zur Induktion eines Entgiftungsenzyms der Phase II, ausgewählt aus Isothiocyanate, Diphenole, Thiocarbamate, 1,2-Dithol-3-thione, Indole-3-Carbinol, Sulforaphan, Brassicaceae-Extrakt, (INCI) Lepidium Sativum sprout extract (Garten Kresse), Xymeninsäure, (INCI) Curcurma Longa root extract, (INCI) Schizandra Chinensis fruit oil, Hemlock-Blatteextrakt, (INCI) Limnanthes alba seed oil, Citrus-Triterpenoide oder (INCI) Ellagic acid und (ii) mindestens ein Bestandteil zur Induktion eines Entgiftungsenzyms der Phase III, ausgewählt aus Eingekapseltes Magnesiumchlorid, eingekapseltes Salz, eingekapseltes Meersalz, Meersalz, eingekapseltes Salz-Extrakt, eingekapseltes Meersalz-Extrakt, (INCI) Hypericum perforatum extract oder (INCI) Camellia Sinensis leaf extract.

## Revendications

1. Une méthode cosmétique pour une élimination améliorée des toxines de la peau et des tissus adipeux sous-cutanés comprenant (i) un traitement d'extraction choisi parmi un enveloppement corporel, un masque, un masque à l'argile, les sels de bain, les bains de boue, les bains, (ib) la transpiration induite par la chaleur, l'exercice, la stimulation électrique, la stimulation mécanique, l'occlusion ou l'iontophorèse inverse et (ii) l'application d'une composition topique comprenant au moins un inducteur de détoxification cellulaire, dans lequel ledit inducteur de détoxification cellulaire est (iia) une substance pour induire une enzyme de détoxication de phase II sélectionnée parmi isothiocyanates, diphénols, thiocarbamates, 1,2-dithol-3-thiones, indole-3-carbinol, Sulforaphane, extrait de Brassicaceae, (INCI) Lepidium Sativum sprout extract (cresson de jardin), acide xyménynique, (INCI) Curcurma Longa root extract, (INCI) Schizandra Chinensis fruit oil, extrait de feuille de pruche , (INCI) Limnanthes alba seed oil, triterpénoïdes d'agrumes ou (INCI) Ellagic acid, ou dans lequel ledit inducteur de détoxification cellulaire est (iib) une substance pour induire une enzyme de détoxication de phase III sélectionnée parmi chlorure de magnésium encapsulé, sel encapsulé, sel de mer encapsulé, sel de mer, extrait de sel encapsulé, extrait de sel de mer encapsulé, (INCI) Hypericum perforatum extract ou (INCI) Camellia Sinensis leaf extract.

2. Un procédé selon l'une quelconque des revendications précédentes, dans lequel au moins un ingrédient de ladite composition est encapsulé par au moins une technique choisie parmi liposomes, microsphères, microémulsion, liposomes enrobés, vectorisation des phospholipides ou micelles.

3. Un procédé selon l'une quelconque des revendications précédentes, dans lequel ledit traitement d'extraction est un enveloppement corporel utilisant des bandages trempés dans une composition d'enveloppement corporel comprenant au moins un ingrédient choisi parmi l'argile, la bentonite, le chlorure de magnésium, le sulfate de magnésium, le chlorure de sodium ou l'oxyde de zinc.

4. Un procédé selon l'une quelconque des revendications précédentes, dans lequel ladite composition est appliquée en utilisant l'iontophorèse par un type de courant choisi parmi de micro-courant, un courant galvanique ou un courant faradique.

5. Un procédé selon l'une quelconque des revendications précédentes, dans lequel ladite composition de détoxification cellulaire comprend au moins un ingrédient supplémentaire sélectionnée parmi acide alpha-lipoïque, N-acétylcystéine, la glutamine, glycine, tocophérol, alkylresorcinols flavonoïdes, (INCI) Malachite extract, (INCI) Sophora Japonica Flower extract, (INCI) Angelica Archangelica Root, (INCI) Silybin, (INCI) Grapefruit extract, (INCI) Olea Europaea Fruit extract, extrait d'agrumes, extrait de son de blé, polyphénols, flavonoïdes, stilbènes, proanthocyanidines, (INCI) quercetin; (INCI) uric acid, l'acide lipoïque, caroténoïdes, (INCI) ferulic acid, (INCI) caffeic acid, Coenzyme Q10, vitamine A, vitamine B, vitamine C, vitamine E, (INCI) Genistein, (INCI) Citrus Grandis Fruit Extract, (INCI) Camellia Sinensis Leaf Extract, (INCI) Vitis Vinifera Seed Extract, (INCI) Silybin, (INCI) Daucus Carota Sativa Root Extract, extrait de Solanum Lycopersicum, (INCI) Coffea Robusta Seed Extract, (INCI) Punica Granatum Seed Extract, (INCI) Niacinamide, (INCI) Pinus Pinaster Bark Extract, (INCI) rutin, (INCI) asiaticoside, acide silicique, flavonoïdes d'agrumes, (INCI) Centella Asiatica Extract, (INCI) Equisetum Arvense Extract, (INCI) Solidago Virgaurea Extract, (INCI) Arnica Montana Flower Extract, (INCI) Vaccinium Macrocarpon Fruit Extract, (INCI) Hedera Helix Extract, (INCI) ruscogenin, (INCI) escin, la forskoline, (INCI) Ruscus Aculeatus Root Extract, (INCI) Coleus Forskohlii Root Extract, (INCI) Vaccinium Myrtillus Fruit/Leaf Extract, (INCI) Aesculus Hippocastanum Extract, xanthines, isoprotérénol, (INCI) tripeptide-3, (INCI) tripeptide-1, L-carnitine, (INCI) synephrine, (INCI) Paullinia Cupana Fruit Extract, (INCI) Citrus Aurantium Amara Peel, (INCI) Cynara Scolymus (Artichoke) Bud Extract, (INCI) Fucus Vesiculosus Extract, extrait d'algues, (INCI) Magnolia Biondii Bark Extract, (INCI) Cola Nitida Seed Extract, (INCI) fisetin, frambinone, (INCI) aspartame, (INCI) resveratrol, Rosmarinic acid, tripeptides de la séquence Val-Tyr-Pro, tripeptides de la séquence Val-Thr-Leu, (INCI) Astragalus Membranaceus Root Extract, (INCI) Nephelium Longana Seed Extract, (INCI) Vitis Vinifera Seed Extract, (INCI) Rosmarinus Officinalis Extract ou (INCI) Nigella Sativa Seed Oil.

6. Un procédé selon l'une quelconque des revendications précédentes, dans lequel ledit procédé est destiné à un usage choisi parmi (i) réduction du volume corporel, (ii) le traitement de la cellulite, (iii) améliorer et raffermir la peau et tissu graisseux sous-cutané, (iv) amélioration du contour du corps (v) l'élimination des toxines de la peau et du corps, (vi) améliorer la perte du poids, (vii) améliorer une méthode d'enveloppement, (viii) améliorer les méthodes minceur cosmétiques.

7. Un procédé selon l'une quelconque des revendications précédentes, dans lequel au moins un principe actif pour induire une enzyme de détoxication de phase II est sélectionnée en fonction d'un facteur choisi parmi occupation, mode de vie, habitudes alimentaires, âge, le genre ou santé générale du sujet.

8. Un composition à utiliser dans des méthodes thérapeutiques pour l'élimination des toxines de la peau et des tissus adipeux sous-cutanés, dans lequel ledit composition de détoxification cellulaire comprenant (i) au moins un ingrédient pour induire une enzyme de détoxication de phase II sélectionnée parmi isothiocyanates, diphénols, thiocarbamates, 1,2-dithol-3-thiones, indole-3-carbinol, Sulforaphane, extrait de Brassicaceae, (INCI) Lepidium Sativum sprout extract (cresson de jardin), acide xyménynique, (INCI) Curcurma Longa root extract, (INCI) Schizandra Chinensis fruit oil, extrait de feuille de pruche , (INCI) Limnanthes alba seed oil, triterpénoïdes d'agrumes ou (INCI) Ellagic acid; et (ii) au moins un ingrédient pour induire une enzyme de détoxication de phase III sélectionnée parmi chlorure de magnésium encapsulé, sel encapsulé, sel de mer encapsulé, sel de mer, extrait de sel encapsulé, extrait de sel de mer encapsulé, (INCI) Hypericum perforatum extract ou (INCI) Camellia Sinensis leaf extract.
